(19) Europäisches Patentamt
European Patent Office
Office européen des brevets

(11) Veröffentlichungsnummer: **0 066 153**
**B1**

# (12) EUROPÄISCHE PATENTSCHRIFT

(45) Veröffentlichungstag der Patentschrift: **03.10.84**

(51) Int. Cl.³: **C 07 C 79/36**, C 07 C 97/10 // C09B29/42

(21) Anmeldenummer: **82104221.5**

(22) Anmeldetag: **14.05.82**

## (54) 4-Isopropyl-4'-nitrobenzophenon und Verfahren zu seiner Herstellung.

(30) Priorität: **25.05.81 DE 3120747**

(43) Veröffentlichungstag der Anmeldung: **08.12.82 Patentblatt 82/49**

(45) Bekanntmachung des Hinweises auf die Patenterteilung: **03.10.84 Patentblatt 84/40**

(84) Benannte Vertragsstaaten: **CH DE FR GB LI**

(56) Entgegenhaltungen:
**DE - A - 2 201 208**

**C. FERRI "Reaktionen der organischen Synthese" 1978, GEORG THIEME VERLAG, Stuttgart Seiten 314 bis 315**
**Chemical Abstracts Band 65, Nr. 13 19. Dezember 1966 K.G. RUTHERFORD et al. "Oxidation of some benzhydrols with bromine in methanol" Abstract Nr. 20046c**

(73) Patentinhaber: **CASSELLA Aktiengesellschaft, Hanauer Landstrasse 526, D-6000 Frankfurt am Main 61 (DE)**

(72) Erfinder: **Tappe, Horst, Dr., Ringstrasse 9, D-6057 Dietzenbach (DE)**
Erfinder: **Wille, Herbert, Dr., Hünfelder Strasse 16, D-6000 Frankfurt am Main 61 (DE)**

(74) Vertreter: **Urbach, Hans-Georg, Dr. et al, Hanauer Landstrasse 526, D-6000 Frankfurt am Main 61 (DE)**

**EP 0 066 153 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft das 4-Isopropyl-4'-nitrobenzophenon sowie ein Verfahren zu seiner Herstellung und seine Verwendung zur Herstellung von 4-Isopropyl-4'-aminobenzophenon.

Aromatische Ketone können nach der Friedel-Craftsschen Ketonsynthese durch Umsetzung einer Acylierungskomponente, in der Regel eines Carbonsäurehalogenids, mit einem aromatischen Kohlenwasserstoff in Gegenwart eines Katalysators hergestellt werden (vgl. z.B. George A. Olah; „Friedel-Crafts- and Related Reaction", vol. III, „Acylation and Related Reaction", Parts 1 and 2, Interscience Publishers New York-London-Sydney [1964]; „Helv. Chim. Acta", *41*, 894 (1958); „Angew. Chem.", *84*, 295 [1972]; „Synthesis" [1978] 672).

Als Lösungsmittel für die Friedel-Craftssche Ketonsynthese sind aus C. Ferri „Reaktionen der organischen Synthese" [1978], S. 314, z.B. Nitrobenzol, Tetrachlorethan, Schwefelkohlenstoff und Chlorbenzol bekannt.

Zum Teil sind bei der Friedel-Craftsschen Ketonsynthese die Ausbeuten niedrig oder das gewünschte Endprodukt muss noch gereinigt werden. So erhält man z.B. bei der Umsetzung von tert.-Butylbenzol, 4-Nitrobenzoylchlorid und Aluminiumchorid ein Rohprodukt, aus dem das gewünschte 4-tert.-Butyl-4'-nitrobenzophenon mühsam durch Umkristallisation gewonnen werden muss. Die Ausbeute beträgt nur 40% („J. Org. Chem.", *31*, 2708 [1966]).

Das 4-Isopropyl-4'-nitrobenzophenon kann leicht in guten Ausbeuten und hohen Reinheiten nach der Friedel-Craftsschen Ketonsynthese synthetisiert werden. Dabei wird Isopropylbenzol (Cumol) mit einem p-Nitrobenzoylhalogenid in Gegenwart eines Friedel-Crafts-Katalysators bei Temperaturen von 10 bis 140°C in einen aliphatischen Kohlenwasserstoff, chlorierten aliphatischen oder chlorierten aromatischen Kohlenwasserstoff umgesetzt.

Als p-Nitrobenzoylhalogenid kann man z.B. das p-Nitrobenzoylbromid oder das p-Nitrobenzoylchlorid verwenden. Das p-Nitrobenzoylchlorid ist bevorzugt. Das Molverhältnis zwischen dem Isopropylbenzol und dem p-Nitrobenzoylhalogenid beträgt bei der Umsetzung normalerweise 1 : (1 bis 1,2), vorzugsweise 1 : (1 bis 1,05).

Beispiele für als Lösungsmittel geeignete aliphatische Kohlenwasserstoffe sind aliphatische Kohlenwasserstoffe mit 6 bis 16 C-Atomen (Hexan bis Hexadecan) oder Gemische davon. In der Regel wird als aliphatischer Kohlenwasserstoff niedrig- oder hochsiedender Petrolether verwendet. Chlorierte aliphatische Kohlenwasserstoffe, die als Lösungsmittel geeignet sind, sind z.B. Chloroform, Tetrachlorkohlenstoff, insbesondere Ethylenchlorid. Chlorierte aromatische Kohlenwasserstoffe, die als Lösungsmittel geeignet sind, sind z.B. Chlorbenzol, o-Dichlorbenzol. Chlorbenzol wird als Lösungsmittel bevorzugt. Es können auch Gemische verschiedener Lösungsmittel benutzt werden.

Bezogen auf die Menge des Isopropylbenzols wird normalerweise die 4- bis 16fache, vorzugsweise die 7- bis 12fache Menge Lösungsmittel eingesetzt.

Als geeignete Katalysatoren werden die aus der Literatur bekannten (vgl. George A. Olah, loc. cit., S. 8 bis 13) Friedel-Crafts-Katalysatoren verwandt, also z.B. Metallhalogenide, wie $AlCl_3$, $FeCl_3$, $SbCl_5$, $SnCl_4$, $TiCl_4$, $BiCl_3$, $AlBr_3$, $FeBr_3$, $SbBr_3$, $ZnBr_2$; Säuren, wie z.B. $HClO_4$, $HOSO_2F$, $H_3PO_4$, $H_3PO_3$, $H_2PO_3F$. Besonders geeignet sind freie oder polymergebundene Perfluoralkansulfonsäuren mit 1 bis 10 C-Atomen in der Perfluoralkankette, ferner $FeCl_3$ und $AlCl_3$. Ganz besonders bevorzugt ist $AlCl_3$. Der Katalysator wird in etwa molarer Menge eingesetzt.

Die Reaktionstemperatur hängt stark von der Art des eingesetzten Katalysators ab. Bei $AlCl_3$ führt man die Reaktion bei 10 bis 70°C durch, wobei man die Reaktion bei niedriger Temperatur startet und bei höherer Temperatur nacherhitzt. Bei $FeCl_3$ oder bei freien oder polymergebundenen Perfluoralkansulfonsäuren liegt der Temperaturbereich bei 50 bis 140°C.

Die drei Reaktionspartner, d.h. das Isopropylbenzol, der Katalysator und das p-Nitrobenzoylhalogenid lassen sich nach 4 Möglichkeiten in dem Lösungsmittel umsetzen:

1. Lösungsmittel + Katalysator + Säurehalogenid vorlegen, dazu das Isopropylbenzol geben;
2. Lösungsmittel + Katalysator + Isopropylbenzol vorlegen, dazu das Säurehalogenid geben;
3. Lösungsmittel + Säurehalogenid + Isopropylbenzol vorlegen, dazu den Katalysator geben;
4. Lösungsmitel + Katalysator vorlegen, dazu das Säurehalogenid + Isopropylbenzol geben.

Bevorzugt werden die 3. und 4. Möglichkeiten, wobei die 4. Möglichkeit besonders bevorzugt ist. Bei dem Vorgehen nach der 4. Möglichkeit kann man zweckmässig das Gemisch von Säurehalogenid und Isopropylbenzol noch mit dem Lösungsmittel verdünnen, um so ein leicht dosierbares, flüssiges Gemisch zu erhalten und um die Reaktion besser kontrollieren zu können.

Das 4-Isopropyl-4'-nitrobenzophenon lässt sich nach bekannten Reduktionsverfahren, wie z.B. mit Wasserstoff, in Gegenwart von Katalysatoren, gegebenenfalls unter Druck, oder mit Eisen oder NaHS in das entsprechende 4-Isopropyl-4'-aminobenzophenon überführen, das ein organisches Zwischenprodukt darstellt, das sich zur Herstellung von Pharmazeutika, Pflanzenschutzmitteln, bevorzugt aber zur Herstellung von Farbstoffen, eignet. Besonders bevorzugt lassen sich aus 4-Isopropyl-4'-aminobenzophenon, z.B. in an sich bekannter Weise durch Diazotierung und Kupplung, wasserunlösliche Farbstoffe der Formel I herstellen

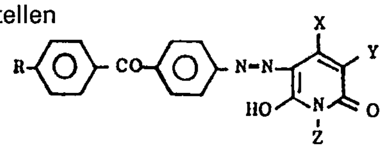

(I)

worin R Isopropyl und X Wasserstoff, Phenyl, Alkyl mit 1 bis 6 C-Atomen, das auch durch Halogen oder Phenyl substituiert sein kann, Y Cyan, Nitro, Alkylcarbonyl mit 1 bis 3 C-Atomen im Alkylrest, Alkoxycarbonyl mit 1 bis 4 C-Atomen im Alkoxyrest, Aminocarbonyl, dessen Aminogruppe auch durch Phenyl monosubstituiert oder durch Alkyl mit 1 bis 4 C-Atomen mono- oder disubstituiert sein kann, Aminosulfonyl, dessen Aminogruppe auch durch Phenyl monosubstituiert oder durch Alkyl mit 1 bis 4 C-Atomen mono- oder disubstituiert sein kann, Phenylsulfonyl, Alkylsulfonyl mit 1 bis 4 C-Atomen in den Alkylresten und Z Wasserstoff, Alkyl mit 1 bis 10 C-Atomen, das auch durch Sauerstoff ein- oder mehrfach unterbrochen und/oder auch durch Phenyl, Phenoxy, Halogen, Hydroxy substituiert sein kann, Alkenyl mit 2 bis 7 C-Atomen, das auch durch Phenyl, Halogen, Hydroxy, Alkoxy mit 1 bis 4 C-Atomen substituiert sein kann, Cycloalkyl mit 5 bis 7 C-Atomen und Phenyl sein kann, bedeuten.

Diese Farbstoffe eignen sich vorzüglich zum Färben und Bedrucken von synthetischem, hydrophobem Fasermaterial, bevorzugt aus Polyester, und für Gemische von Cellulosefasern mit den synthetischen, organischen Fasern nach Auszieh-, Thermosol- und Druckverfahren.

Das 4-Isopropyl-4'-nitrobenzophenon kann nach der vorliegenden Erfindung in einfacher Weise ohne umständliche Reinigungsoperationen in guter Reinheit und mit Ausbeuten bis zu ca. 95% der Theorie erhalten werden.

Sofern nichts anderes angegeben, bedeuten Teile in den nachfolgenden Beispielen Gewichtsteile.

*Beispiel 1*

710 Teile Chlorbenzol und 160 Teile $AlCl_3$ werden vorgelegt. Dazu tropft man im Verlauf von 1 h bei 20 bis 25°C eine Mischung aus 120 Teilen Cumol, 185,6 Teilen p-Nitrobenzoylchlorid und 410 Teilen Chlorbenzol, wobei HCl-Gas frei wird. Man heizt dann im Verlauf von 1 h auf 60°C, rührt 1 h bei 60°C, gibt 1500 Teile Eis zu, trennt die wässerige Phase im Scheidetrichter ab und engt im Vakuum ein. Man erhält 247,6 Teile 4-Isopropyl-4'-nitrobenzophenon vom Schmelzpunkt 95°C. Ausbeute: 92% der Theorie.

Die Reduktion des 4-Isopropyl-4'-nitrobenzophenons kann z.B. wie folgt durchgeführt werden:

800 Teile Wasser, 250 Teile Eisenspäne und 28 Teile Essigsäure werden vorgelegt, auf 95°C erhitzt und 1 h bei 95°C gerührt. Dann kühlt man auf 90°C ab, gibt 800 Teile Ethanol zu und trägt in 30 min 55 Teile 4-Isopropyl-4'-nitrobenzophenon in fester Form in kleinen Portionen ein. Es wird 8 h am Rückfluss erhitzt, mit 44 Teilen Soda auf pH 9 gestellt, 600 Teile Ethanol zugegeben, 15 h am Rückfluss erhitzt, heiss filtriert und der Rückstand dreimal mit je 200 Teilen heissem Ethanol gewaschen. Aus den vereinigten Filtraten wird das Ethanol abdestilliert, und der wässerige Sumpf wird abgekühlt. Anschliessend filtriert man die ausgefallenen Kristalle ab und trocknet im Vakuum. Man erhält 45,5 Teile 4-Isopropyl-4'-aminobenzophenon vom Schmelzpunkt 110°C. Aus dem 4-Isopropyl-4'-aminobenzophenon lässt sich, wie in der DE-OS Nr. 2001821, Tabelle 1, Beispiel 15, angegeben, der wertvolle Farbstoff der Formel II

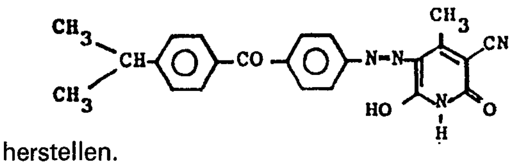

(II)

herstellen.

*Beispiel 2*

1100 Teile Chlorbenzol, 120 Teile Cumol und 185,6 Teile p-Nitrobenzoylchlorid werden vorgelegt. Im Verlauf von 1 h trägt man bei 20 bis 25°C 160 g $AlCl_3$ ein, wobei die HCl-Entwicklung einsetzt. Verfährt man weiter wie in Beispiel 1 angegeben, so erhält man 246 Teile 4-Isopropyl-4'-nitrobenzophenon. Ausbeute: 91,4% der Theorie.

*Beispiel 3*

1200 Teile Ethylenchlorid, 120 Teile Cumol und 185 Teile p-Nitrobenzoylchlorid werden vorgelegt und im Verlauf von 1 h bei 25 bis 30°C mit 160 g $AlCl_3$ versetzt. Verfährt man im übrigen wie im Beispiel 1 angegeben, so erhält man 226 Teile 4-Isopropyl-4'-nitrobenzophenon. Ausbeute: 84% der Theorie.

## Patentansprüche

1. 4-Isopropyl-4'-nitrobenzophenon.

2. Verfahren zur Herstellung von 4-Isopropyl-4'-nitrobenzophenon, dadurch gekennzeichnet, dass Isopropylbenzol mit einem p-Nitrobenzoylhalogenid in Gegenwart eines Friedel-Crafts-Katalysators bei Temperaturen von 10 bis 140°C in einem aliphatischen Kohlenwasserstoff, chlorierten aliphatischen oder chlorierten aromatischen Kohlenwasserstoff umgesetzt wird.

3. Verwendung von 4-Isopropyl-4'-nitrobenzophenon zur Herstellung von 4-Isopropyl-4'-aminobenzophenon.

## Claims

1. 4-isopropyl-4'-nitrobenzophenone.

2. Process for the preparation of 4-isopropyl-4'-nitrobenzophenone characterised in that isopropylbenzene is reacted with a p-nitrobenzoyl halide in the presence of a Friedel-Crafts catalyst at temperatures of 10 to 140°C in an aliphatic hydrocarbon, or a chlorinated aliphatic or chlorinated aromatic hydrocarbon.

3. Use of 4-isopropyl-4'-nitrobenzophenone for the preparation of 4-isopropyl-4'-aminobenzophenone.

## Revendications

1. Isopropyl-4-nitro-4'-benzophénone.

2. Procédé de préparation de l'isopropyl-4-nitro-4'-benzophénone, procédé caractérisé en ce qu'on fait réagir l'isopropylbenzène avec un halogénure de p-nitrobenzoyle, en présence d'un catalyseur de Friedel-Crafts, à des températures de 10 à 140°C, dans un hydrocarbure aliphatique, un hydrocarbure aliphatique chloré ou un hydrocarbure aromatique choré.

3. Application de l'isopropyl-4-nitro-4'-benzophénone à la préparation de l'isopropyl-4-amino-4'-benzophénone.